# EUROPÄISCHE PATENTSCHRIFT

(11) **EP 3 801 670 B1**
(45) Veröffentlichungstag und Bekanntmachung des Hinweises auf die Patenterteilung: **13.07.2022**
(21) Anmeldenummer: 19730288.8
(22) Anmeldetag: 06.06.2019
(51) Int. Cl.: A61M 60/135, A61M 60/148, A61M 60/857

(54) **LEITUNGSVORRICHTUNG FÜR EIN HERZUNTERSTÜTZUNGSSYSTEM UND VERFAHREN ZUM HERSTELLEN EINER LEITUNGSVORRICHTUNG**
LINE DEVICE FOR A VENTRICULAR ASSIST DEVICE AND METHOD FOR PRODUCING A LINE DEVICE
SYSTÈME DE LIGNE ÉLECTRIQUE POUR DISPOSITIF D'ASSISTANCE VENTRICULAIRE ET PROCÉDÉ POUR RÉALISER LE SYSTÈME DE LIGNE ÉLECTRIQUE

(30) Priorität: 06.06.2018 DE 102018208911
(43) Veröffentlichungstag der Anmeldung: 14.04.2021
(73) Patentinhaber: Kardion GmbH, 70376 Stuttgart (DE)
(72) Erfinder: SCHLEBUSCH, Thomas, Alexander, 71272 Renningen (DE); MINZENMAY, David, 70569 Stuttgart (DE); KASSEL, Julian, 75181 Pforzheim (DE); BAECHLE, Tobias, 70191 Stuttgart (DE)
(74) Vertreter: Pfiz, Thomas
(86) Internationale Anmeldenummer: PCT/EP2019/064775
(87) Internationale Veröffentlichungsnummer: WO 2019/234146

(56) Entgegenhaltungen:
- WO-A1-2017/147291
- WO-A1-2019/229220
- US-A1- 2008 015 517
- US-A1- 2013 289 376
- US-A1- 2015 190 092
- US-A1- 2016 144 166
- US-A1- 2018 064 860

## Beschreibung

Die Erfindung geht von einer Leitungsvorrichtung oder einem Verfahren zum Herstellen einer Leitungsvorrichtung nach Gattung der unabhängigen Ansprüche aus. Gegenstand der vorliegenden Erfindung ist auch ein Computerprogramm.

Durch erhebliche Fortschritte in den Materialwissenschaften ist mittlerweile eine Herstellung dünner, flexibler und gleichzeitig komplex strukturierter elektrischer Leiterstrukturen möglich, wie beispielsweise die Veröffentlichung Burkard et al.: Flex Technology for Foldable Medical Flip Chip Devices; IMAPS Conf. on Device Packaging, Scottdale AZ, March 17-20, 2008 beschreibt. Solche elektrischen Leiterstrukturen kommen im Bereich der Medizintechnik beispielsweise in Form von implantierten Augen-Innendrucksensoren oder Retina-Implantaten zum Einsatz.

Ausgehend hiervon liegt der Erfindung die Aufgabe zugrunde, eine in ihrer Integration und Funktionalität vereinfachte und verbesserte Leitungsvorrichtung für ein Herzunterstützungssystem sowie ein vorteilhaftes Verfahren zu deren Herstellung anzugeben.

Vor diesem Hintergrund werden mit dem hier vorgestellten Ansatz eine Leitungsvorrichtung für ein Herzunterstützungssystem, ein Verfahren zum Herstellen einer Leitungsvorrichtung, weiterhin eine Vorrichtung, die dieses Verfahren verwendet, sowie schließlich ein entsprechendes Computerprogramm gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Die hier vorgestellte Leitungsvorrichtung für ein Herzunterstützungssystem beschreibt ein hochfrequenztaugliches elektrisches Leitelement beispielsweise auf Basis eines flexiblen Substrats, das die Funktionen eines Sensorträgers, einer elektrischen Verbindungsleitung sowie eines Anschlusselements in einer einzelnen Baugruppe integrieren kann, wodurch beispielsweise zusätzliche Verbindungsstellen auf der Pumpe des Herzunterstützungssystems vermieden werden können. Hierdurch kann der Produktionsprozess vereinfacht und die Zuverlässigkeit des Herzunterstützungssystems gesteigert werden.

Es wird eine Leitungsvorrichtung für ein Herzunterstützungssystem vorgestellt, wobei die die erfindungsgemäße Leitungsvorrichtung die Merkmale des Anspruchs 1 aufweist.

Bei einer Leitungsvorrichtung kann es sich um ein Bauelement eines Herzunterstützungssystems handeln, das der Integration eines hochfrequenztauglichen elektrischen Leitelements, beispielsweise innerhalb einer Führungskanüle der Leitungsvorrichtung, dient. Unter einem Herzunterstützungssystem, auch Kunstherz oder VAD (ventricular assist device) genannt, kann eine Pumpeinrichtung zur Steigerung der Pumpleistung eines Herzens verstanden werden. Das Herzunterstützungssystem kann beispielsweise mittels Katheter in eine Herzkammer oder die Aorta einführbar sein. Insbesondere kann es sich bei dem Herzunterstützungssystem um ein linksventrikuläres Unterstützungssystem handeln, welches beispielsweise auch als perkutanes Unterstützungssystem ausgestaltet sein kann aber nicht zwingend sein braucht. Bei einer Führungskanüle kann es sich um ein zylinderförmiges Gehäuse handeln, das beispielsweise eine metallhaltige Legierung und/oder einen konstanten Außendurchmesser aufweisen kann, alternativ jedoch auch eine Verjüngung aufweisen kann. Somit kann die Führungskanüle der Aufnahme eines elektrischen Leitelements bzw. einer elektrischen Verbindungsleitung dienen und beispielsweise Anwendung in einer Leitungsvorrichtung eines Herzunterstützungssystems finden. Die Führungskanüle kann ferner eine strukturierte Oberfläche oder Strukturen in einem Mantel aufweisen, die beispielsweise als ein Geflecht und/oder als eine aus einem Rohr geschnittene Spiral- oder Wellenstruktur oder als gezackte Struktur oder als Zickzack-Variante ausgeformt sein kann. Unter einem elektrischen Leitelement kann eine elektrische Verbindungsleitung verstanden werden, die beispielsweise innerhalb einer Führungskanüle eines Herzunterstützungssystems angeordnet ist und der elektrischen Verbindung einer Sensorik, beispielsweise eines Druck- und/oder Temperatursensors, in einer distalen Spitze des Herzunterstützungssystems mit einem elektrischen Anschlusskabel an einem proximalen Ende besagten Herzunterstützungssystems dienen kann. Bei einer Mehrschichtstruktur kann es sich um einen mehrschichtigen Strukturaufbau eines elektrischen Leitelements und/oder elektrischen Verbindungskabels handeln, wobei jede einzelne Schicht eine spezifische Funktionalität, beispielsweise eine leitende und/oder eine isolierende Funktion, bieten kann. So kann die Mehrschichtstruktur beispielsweise mittels eines Dünnschichtverfahrens gefertigt werden.

Die Vorteile des hier vorgestellten Ansatzes einer Leitungsvorrichtung für ein Herzunterstützungssystem liegen beispielsweise darin, dass ein elektrisches Leitsystem der Leitungsvorrichtung insbesondere unter Verwendung eines Dünnschichtverfahrens realisiert wird, wobei das Dünnschichtverfahren eine Reduktion eines Dickenauftrags im Vergleich zu einem klassischen elektrischen Verbindungskabel bietet. So kann das elektrische Leitelement ferner beispielsweise einstückig realisiert sein und zusätzlich beispielsweise die Funktionen eines Sensorträgers, einer elektrischen Verbindungsleitung sowie eines Anschlusselements in einer einzelnen Baugruppe vereinen, wobei diese Form der Realisierung mögliche Fehlerstellen reduziert und darüber hinaus unnötige Kontaktstellen vermieden werden, die einen zusätzlichen Dickenauftrag darstellen würden. Zur Vermeidung von Kontaktstellen wird ferner beispielsweise ein einziges (beispielsweise einstückiges) flexibles Substrat sowohl in der Sensorkopfeinheit, als Führung des elektrischen Leitelements entlang der Führungskanüle sowie auch zur elektrischen Kontaktierung an ein Durchführungselement an der Endeinheit des Herzunterstützungssystems verwendet. Dieses flexible Substrat, beispielsweise ein Dünnschichtsubstrat, kann durch einen Kleber vorfixiert werden und anschließend mit einer Schutzlackschicht überzogen sein, die einen Schutz der Leitungsvorrichtung gegen eine mögliche Beschädigung herstellt.

Gemäß einer Ausführungsform kann das elektrische Leitelement eine Mehrzahl von (beispielsweise koplanaren) Schichten aus einem leitenden und/oder isolierenden Material aufweisen, insbesondere wobei eine leitende Schicht zumindest teilweise ein Goldmaterial aufweist und/oder eine isolierende Schicht zumindest teilweise aus einem Polyimid-Material gefertigt ist. Eine solche Ausführungsform des hier vorgestellten Ansatzes bietet den Vorteil, dass aus der Kombination verschiedener Schichten zum Aufbau eines elektrischen Leitelements sich beispielsweise neue und/oder verbesserte Eigenschaften und Anwendungsfelder des elektrischen Leitelements ergeben können. So kann das elektrische Leitelement beispielsweise unter Verwendung eines Dünnschichtverfahrens gefertigt werden, wobei eine Realisierung des elektrischen Leitelements im Dünnschichtverfahren vorteilhaft eine Reduktion des Dickenauftrags bietet. Ferner ermöglicht eine Herstellung solcher Schichten beispielsweise mittels waferbasierter Lithografieprozesse die Realisierung ressourcen- und energieeffizienter Fertigungsprozesse einer Leitungsvorrichtung. Die Schichten werden beispielsweise durch Lithografie (insbesondere durch Aufbringen von Fotoresist, Belichten, Entwickeln, Grundschicht sputtern, galvanisch Aufdicken, Fotoresist entfernen) hergestellt.

Polyimid-Materialien werden in der Elektrotechnik beispielsweise aufgrund ihrer Hitzebeständigkeit, geringen Ausgasung, Strahlungsbeständigkeit und Isoliereigenschaften in Form von hellbräunlichen, halbtransparenten Folien zur Anwendung gebracht. Hohe Dauereinsatztemperaturen von bis zu 230 °C und kurzzeitig bis 400 °C sind hierbei möglich. Polyimid-Materialien können beispielsweise insbesondere für besonders dünne und dennoch recht stabile Lackisolierungen von elektrischen Leitungen im Dünnschichtverfahren eingesetzt werden. Besonders vorteilhaft ist der mehrschichtige Aufbau eines Leiters auf beispielsweise einem Glas-Trägersubstrat auf Basis von Polyimid, dass dieses in flüssiger Form mittels Spin Coating aufgebracht werden kann. Im Gegensatz zu unter Zuhilfenahme eines Klebers auflaminierter Polyimidschichten (wie in der flexiblen Leiterkartenindustrie üblich), ermöglicht die flüssige Isolierlagenherstellung einen hermetischen Einschluss des metallischen Leiters, sodass keine Feuchtigkeit eindringen kann und Korrosionsprobleme reduziert werden. Im Bereich der Medizintechnik wird Polyimid aufgrund seiner Biokompatibilität bevorzugt. Ein Gold-Material bietet den Vorteil, dass es keine Oxydschicht bildet und dadurch stets guter elektrischer Kontakt gewährleistet ist. Besonders hervorzuheben ist die exzellente Biokompatibilität. Weitere denkbare Metalle sind Platin-Iridium oder aufgrund seiner hohen Leitfähigkeit und des geringen Preises prinzipiell auch Kupfer.

Gemäß einer Ausführungsform kann das elektrische Leitelement ein Schirmelement aufweisen, insbesondere wobei das Schirmelement unter Verwendung der leitenden Schichten und/oder einer Durchkontaktierung zwischen den einzelnen Schichten realisiert ist. Die Schirmung kann hierbei beispielsweise durch metallische Lagen sowie flächige Durchkontaktierungen zwischen den einzelnen Lagen des elektrischen Leitelements hergestellt werden. Eine solche Ausführungsform des hier vorgestellten Ansatzes bietet den Vorteil, dass die Schirmung eine Verbesserung der Hochfrequenzeigenschaften (beispielsweise in Bezug auf eine Impedanzkontrolle) der Leitungsvorrichtung bieten kann.

Gemäß einer Ausführungsform kann das elektrische Leitelement eine Mehrzahl von Leitungen aufweisen, wobei einige der Leitungen innerhalb einer Schicht angeordnet sind, insbesondere wobei die Mehrzahl von Leitungen außerhalb des Schirmelements angeordnet ist. Eine solche Ausführungsform des hier vorgestellten Ansatzes bietet den Vorteil, dass das Schirmelement, welches beispielsweise ein metallisches Material aufweisen kann, im Kontaktierungsbereich des elektrischen Leiterelements sehr einfach durch Prozesse gebildet werden kann, die auch zur Herstellung von Leiterbahnen oder Leitungen in dem Leitelement verwendet werden.

Das elektrische Leitelement weist zur Kontaktierung zumindest eines Sensors einen Sensorkontaktbereich auf, und kann ferner, gemäß einer Ausführungsform, zur Kontaktierung zumindest eines Signalgebers einen Signalgeberkontaktierbereich aufweisen. Hierbei kann es sich bei dem zumindest einen Sensor beispielsweise um einen Temperatursensor, der eine Bluttemperatur eines herzkranken Patienten misst und/oder um einen (beispielsweise barometrischen) Drucksensor zur Erfassung eines Ventrikeldrucks eines herzkranken Patienten handeln. Bei dem Signalgeber kann es sich beispielsweise um ein Ultraschallelement handeln, das eine Volumenstrommessung des Blutes eines herzkranken Patienten ermöglicht. Eine solche Ausführungsform des hier vorgestellten Ansatzes bietet den Vorteil, dass durch eine solche Realisierung eines Endes des elektrischen Leitelements sowohl eine Kontaktierung der Sensoren, als auch des Ultraschallelements ermöglicht wird.

Gemäß einer Ausführungsform kann der Sensorkontaktbereich ausgebildet sein zumindest zwei Sensoren aufzunehmen und/oder zu kontaktieren und/oder der Sensorkontaktbereich rechteckig ausgeformt sein, insbesondere wobei der Sensorkontaktbereich zumindest zwei Kanten aufweist, wobei der Sensorkontaktbereich an den zumindest zwei Kanten gebogen ist. Eine solche Ausführungsform des hier vorgestellten Ansatzes bietet den Vorteil, dass der Sensorkontaktbereich an den zwei Kanten gebogen wird, um sich um eine Nut in der Sensorkopfeinheit des Herzunterstützungssystems zu legen und hierdurch einen stabilen und dauerhaften Halt zu gewährleisten. Diese Nut bzw. Sensorkavität kann (beispielsweise nach Einbettung eines Sensors in diese Nut) mit einer Vergussmasse, beispielsweise ein festes und/oder gelartiges Silikon, zum Schutz der Sensoren vor Blut und mechanischer Beschädigung ausgefüllt sein. In einer besonderen Ausführungsform können die geraden Bereiche zwischen den Biegekanten durch Versteifungselemente verstärkt sein, sodass eine Biegung nur im Bereich der Biegekante möglich ist.

Gemäß einer Ausführungsform kann der Signalgeberkontaktierbereich zumindest zwei gebogene Kontaktstellen aufweisen. Der Signalgeberkontaktierbereich ist beispielhaft als eine kreisrunde Leiterplatte ausgebildet, wobei die zumindest zwei gebogenen Kontaktstellen ausgebildet sind, um zumindest einen Signalgeber, beispielsweise ein Ultraschallelement, aufzunehmen bzw. zu kontaktieren. Ferner weist der Signalgeberkontaktierbereich ebenfalls eine Kante auf. Eine solche Ausführungsform des hier vorgestellten Ansatzes bietet den Vorteil, dass der Signalgeberkontaktierbereich ebenfalls an der Kante gebogen werden kann, um sich optimal in die zylinderförmige Ausformung des Herzunterstützungssystems zu integrieren.

Gemäß einer Ausführungsform kann das elektrische Leitelement ein Anschlussstellenelement aufweisen, wobei das Anschlussstellenelement kreisförmig, sechseckig, viereckig, dreieckig, allgemein polygonförmig oder U-förmig ausgeformt ist, insbesondere wobei das Anschlussstellenelement eine Mehrzahl von runden Verbindungsstellen und/oder an einer Außenumgebung des Anschlusselements radial und/oder umlaufend angeordnete Verbindungsstellen aufweist. Eine solche Ausführungsform des hier vorgestellten Ansatzes bietet den Vorteil, dass die halbkreisförmig ausgeformten Verbindungsstellen die dünnen und/oder flexiblen Leitungen des elektrischen Leitelements zwischen die ebenfalls radial und/oder umlaufend angeordneten Kontaktstifte eines Durchführungselements geklappt werden können, um dort mittels Schweißen, Leitkleben oder Löten elektrisch kontaktiert zu werden. Die Form des Anschlussstellenelements als Kreis mit halbkreisförmigen Verbindungsstellen ermöglicht eine komfortable Kontaktierung, wobei selbst ein geringer Längenausgleich durch diese Realisierung umsetzbar ist.

Gemäß einer Ausführungsform kann ein strukturierter Mantel der Führungskanüle als ein Geflecht und/oder als eine aus einem Rohr geschnittene Spiral- /Wellen- oder Zick-Zack-Struktur ausgeformt sein, insbesondere wobei die Führungskanüle eine metallhaltige Legierung aufweist. Eine solche Ausführungsform des hier vorgestellten Ansatzes bietet den Vorteil, dass mittels einer geflochtenen und/oder spiralförmig ausgeformten Struktur der Führungskanüle das elektrische Leitelement mechanisch geschützt und/oder gestützt ist. Wird das Kabel bzw. das Leitelement ohne weitere Vorrichtungen in einen geflochtenen Schlauch integriert, wirken hohe Biegebelastungen auf das Kabel, was vor dem Zeithorizont einer Dauerimplantation zum Bruch der elektrischen Verbindung führen kann. Daher ist es vorteilhaft das Kabel auf einer Stütz- oder Schutzstruktur vorzumontieren, beispielsweise einem metallischen Band, und dieses im Folgenden in das Geflecht zu integrieren. Wird die Führungskanüle aus einem Rohr geschnitten, kann die Form einer Stütz- oder Schutzstruktur im Schneidprogramm integriert werden, wodurch kein separates Bauteil benötigt wird. Insbesondere eine Führung des elektrischen Leitelements über einen spiralförmig ausgeformten Steg einer Führungskanüle bietet dem elektrischen Leitelement einen sehr großen Schutz gegen eine mechanische Dauerbiegebelastung.

Gemäß einer Ausführungsform kann je eine Schicht des elektrischen Leitelements eine Dicke in einem Bereich zwischen 5 µm und 15 µm aufweisen und/oder der elektrische Leiter mäanderförmig ausgeformt sein. Jede Schicht Polyimid (PI) oder Gold kann beispielsweise ca. 5 bis 15µm dick sein. Eine Gesamtdicke des elektrischen Leitelements ist dann abhängig von Anzahl der Lagen und Dicke der Einzellagen, wobei eine Anzahl der Lagen auch von einer vorhandenen Schirmung bzw. Schirmungslage abhängig ist. Beispielsweise kann minimal ein 3-Lagen-System (PI, Gold, PI) eine Dicke von 15 µm aufweisen. Maximal kann beispielsweise ein solches System mit Schirmung eine Dicke von 11 Lagen á max. 10 µm, also 110 µm aufweisen. Eine solche Ausführungsform des hier vorgestellten Ansatzes bietet den Vorteil, dass mittels eines mäanderförmig ausgelegten elektrischen Leitelements ein Längenausgleich des Leitelements bei einer Dehnung oder Stauchung realisiert werden kann.

Der hier vorgestellte Ansatz schafft ferner ein Herzunterstützungssystem mit einer Leitungsvorrichtung entsprechend einer hier vorgestellten Variante, wobei die Leitungsvorrichtung zwischen einer Sensorkopfeinheit und einer Endeinheit des Herzunterstützungssystems angeordnet ist, insbesondere wobei zwischen der Leitungsvorrichtung und der Sensorkopfeinheit und/oder zwischen der Leitungsvorrichtung und der Endeinheit je ein Verbindungselement angeordnet ist. Auch durch eine solche Ausführungsform können die besonderen Vorteile des hier vorgestellten Ansatzes einfach und kostengünstig realisiert werden. Ein erfindungsgemäßes Herzunterstützungssystem weist die Merkmale des Anspruchs 11 auf.

Ein erfindungsgemäßes Verfahren zum Herstellen einer Leitungsvorrichtung weist die Merkmale des Anspruchs 12 auf. Das weist die folgenden Schritte auf:
- Bereitstellen der Führungskanüle sowie des elektrischen Leitelements; und
- Anordnen des elektrischen Leitelements innerhalb der Führungskanüle, um die Leitungsvorrichtung herzustellen.

Das hier vorgestellte Verfahren zum Herstellen einer Leitungsvorrichtung für ein Herzunterstützungssystem kann beispielsweise in Software oder Hardware oder in einer Mischform aus Software und Hardware beispielsweise in einem Steuergerät implementiert sein.

Der hier vorgestellte Ansatz schafft ferner eine Vorrichtung, die ausgebildet ist, um die Schritte einer Variante des hier vorgestellten Verfahrens zum Herstellen einer Leitungsvorrichtung für ein Herzunterstützungssystem in entsprechenden Einrichtungen durchzuführen, anzusteuern bzw. umzusetzen. Eine solche erfindungsgemäße Vorrichtung umfasst die Merkmale des Anspruchs 13. Auch durch dieser Vorrichtung kann die der Erfindung zugrunde liegende Aufgabe schnell und effizient gelöst werden.

Hierzu kann die Vorrichtung zumindest eine Recheneinheit zum Verarbeiten von Signalen oder Daten, zumindest eine Speichereinheit zum Speichern von Signalen oder Daten, zumindest eine Schnittstelle zu einem Sensor oder einem Aktor zum Einlesen von Sensorsignalen von dem Sensor oder zum Ausgeben von Daten- oder Steuersignalen an den Aktor und/oder zumindest eine Kommunikationsschnittstelle zum Einlesen oder Ausgeben von Daten aufweisen, die in ein Kommunikationsprotokoll eingebettet sind. Die Recheneinheit kann beispielsweise ein Signalprozessor, ein Mikrocontroller oder dergleichen sein, wobei die Speichereinheit ein Flash-Speicher, ein EEPROM oder eine magnetische Speichereinheit sein kann. Die Kommunikationsschnittstelle kann ausgebildet sein, um Daten drahtlos und/oder leitungsgebunden einzulesen oder auszugeben, wobei eine Kommunikationsschnittstelle, die leitungsgebundene Daten einlesen oder ausgeben kann, diese Daten beispielsweise elektrisch oder optisch aus einer entsprechenden Datenübertragungsleitung einlesen oder in eine entsprechende Datenübertragungsleitung ausgeben kann.

Unter einer Vorrichtung kann vorliegend ein elektrisches Gerät verstanden werden, das Sensorsignale verarbeitet und in Abhängigkeit davon Steuer- und/oder Datensignale ausgibt. Die Vorrichtung kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen der Vorrichtung beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einer Vorrichtung ausgeführt wird. Ein erfindungsgemäßes Computerprogramm umfasst die Merkmale des Anspruchs 14.

Ausführungsbeispiele des hier vorgestellten Ansatzes sind in den Zeichnungen dargestellt und in der nachfolgenden Beschreibung näher erläutert. Es zeigt:
- Fig. 1: eine schematische Ansicht eines linksventrikulären Herzunterstützungssystems mit einer integrierten Leitungsvorrichtung gemäß einem Ausführungsbeispiel;
- Fig. 2: eine schematische Ansicht einer Sensorkopfeinheit eines linksventrikulären Herzunterstützungssystems gemäß einem Ausführungsbeispiel;
- Fig. 3: eine schematische Ansicht eines Sensorkontaktbereichs sowie eines Signalgeberkontaktierbereichs eines elektrischen Leitelements gemäß einem Ausführungsbeispiel;
- Fig. 4: eine dreidimensionale Ansicht einer Sensorkopfeinheit eines linksventrikulären Herzunterstützungssystems gemäß einem Ausführungsbeispiel;
- Fig. 5: eine schematische Ansicht einer Sensorkopfeinheit eines linksventrikulären Herzunterstützungssystems gemäß einem Ausführungsbeispiel;
- Fig. 6: eine schematische Ansicht einer Führungskanüle einer Leitungsvorrichtung gemäß einem Ausführungsbeispiel;
- Fig. 7: eine schematische Ansicht eines Anschlussstellenelements eines elektrischen Leitelements gemäß einem Ausführungsbeispiel;
- Fig. 8: eine schematische Ansicht eines kontaktierten Anschlussstellenelements eines elektrischen Leitelements gemäß einem Ausführungsbeispiel;
- Fig. 9: eine schematische Querschnittsansicht eines elektrischen Leitelements gemäß einem Ausführungsbeispiel; und
- Fig. 10: ein Ablaufdiagramm eines Ausführungsbeispiels eines Verfahrens zum Herstellen einer Leitungsvorrichtung gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

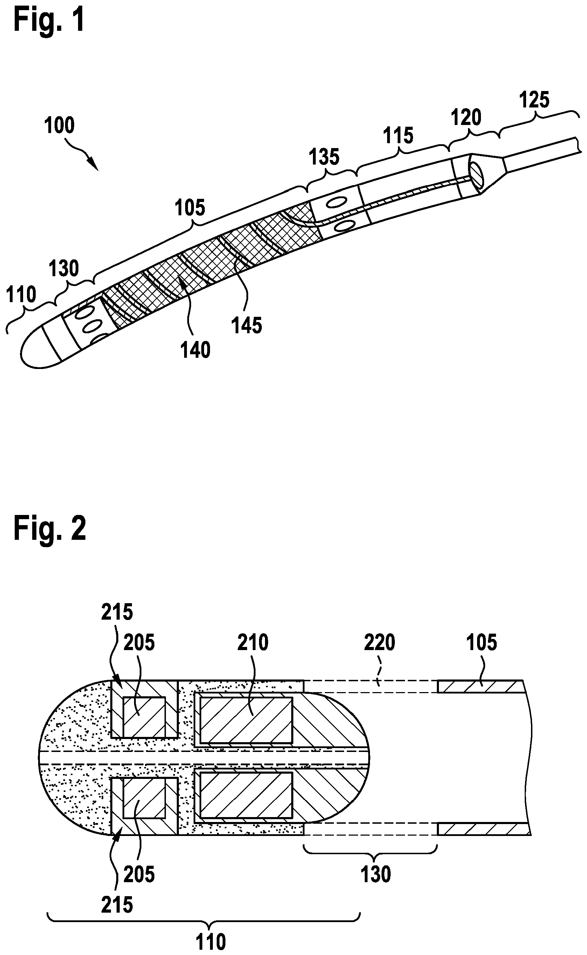

Fig. 1 zeigt eine schematische Ansicht eines linksventrikulären Herzunterstützungssystems 100 mit einer integrierten Leitungsvorrichtung 105 gemäß einem Ausführungsbeispiel. Das Herzunterstützungssystem 100 weist einen zylinderförmigen, länglichen Aufbau mit im Wesentlichen konstantem Außendurchmesser und abgerundeten, sich verjüngenden Enden zur einfachen Platzierung mittels Katheter in einem Blutgefäß, etwa der linken Herzkammer oder der Aorta, auf.

Das Herzunterstützungssystem 100, hier beispielhaft ein linksventrikuläres Herzunterstützungssystem 100 zur perkutanen Implantierung in eine linke Herzkammer, weist zunächst die Leitungsvorrichtung 105 auf, wobei die Leitungsvorrichtung 105 zwischen einer Sensorkopfeinheit 110 sowie einem Motorgehäuse 115, einer Endeinheit 120 und einem Anschlusskabel 125 des Herzunterstützungssystems 100 angeordnet ist. Hierbei kann die Leitungsvorrichtung über je ein Verbindungselement 130 und 135 mit der Sensorkopfeinheit 110 und dem Motorgehäuse 115 bzw. der Endeinheit 120 verbunden sein. Die Verbindungselemente 130 und 135 enthalten Öffnungen zur Aufnahme bzw. Abgabe des Blutes. Die Ankopplung erfolgt beispielsweise durch Kleben. Die Leitungsvorrichtung 105 und das Verbindungselement 130 können auch aus einem Teil bestehen, also einstückig gefertigt sein. Auch kann die Sensorkopfeinheit 110 und das Verbindungselement 130 in einer Ausführung aus einem Teil, also einstückig, ausgeführt sein.

Die Sensorkopfeinheit 110 des Herzunterstützungssystems 100 weist beispielhaft eine Spitze in Form einer Sensorbaugruppe auf, die beispielsweise der Druck- und/oder der Temperaturmessung dient. Die Endeinheit 120 repräsentiert beispielhaft ein proximales Ende des Herzunterstützungssystems 100 und bildet einen Übergang zwischen dem Motorgehäuse 115 des Herzunterstützungssystems 100 und dem Anschlusskabel 125 zum Anschließen des Herzunterstützungssystems 100 an eine externe Energiequelle oder eine externe Auswerte- oder Steuereinrichtung.

Die Leitungsvorrichtung 105 weist eine Führungskanüle 140 auf, die zumindest teilweise entlang einer Erstreckungsrichtung eine Struktur bzw. hier strukturierte Oberfläche aufweist. Beispielshaft weist die Führungskanüle 140 eine spiralförmige Oberflächenstruktur auf. Innerhalb der Führungskanüle 140 ist ein elektrisches Leitelement 145 angeordnet, wobei dieses elektrische Leitelement 145 der elektrischen Verbindung der Sensorkopfeinheit 110 mit dem Anschlusskabel 125 am proximalen Ende des Herzunterstützungssystems 100 dient.

Gemäß einem Ausführungsbeispiel kann das elektrische Leitelement 145 einen Mäander enthalten, um einen Längenausgleich desselbigen zu realisieren. Der Mäander wird hierbei vorzugsweise im Bereich des Motorgehäuses 115 platziert.

Fig. 2 zeigt eine schematische Ansicht einer Sensorkopfeinheit 110 eines linksventrikulären Herzunterstützungssystems 100 gemäß einem Ausführungsbeispiel.

Die Sensorkopfeinheit 110 des Herzunterstützungssystems 100 weist beispielhaft eine Spitze in Form einer Sensorbaugruppe auf, die beispielsweise der Druck- und/oder der Temperaturmessung eines herzkranken Patienten dient. Hierfür weist die Sensorkopfeinheit 110 gemäß einem Ausführungsbeispiel zwei Sensoren 205, sowie einen Signalgeber 210 auf. Bei den zwei Sensoren 205 kann es sich beispielhaft um einen Drucksensor und/oder einen Temperatursensor handeln. Bei dem Signalgeber 210 kann es sich beispielhaft um ein Ultraschallelement handeln. Beide Sensoren 205 sind gemäß einem Ausführungsbeispiel in einer Sensorkavität 215 angeordnet, die mit einer Vergussmasse zum Schutz der Sensoren 205 vor Blut und/oder einer mechanischen Beschädigung verfüllt ist. So kann es sich beispielhaft bei dieser Vergussmasse um ein festes und/oder gelartiges Silikon und/oder ein Silikonöl handeln.

Wie in der hier gezeigten schematischen Ansicht einer Sensorkopfeinheit 110 dargestellt, ist die Sensorkopfeinheit 110 über das Verbindungselement 130 mit der Leitungsvorrichtung 105 verbunden, wobei das Verbindungselement 130 eine Mehrzahl von Zulauffenstern 220 aufweist, über die das Blut des herzkranken Patienten in das Herzunterstützungssystem eintritt.

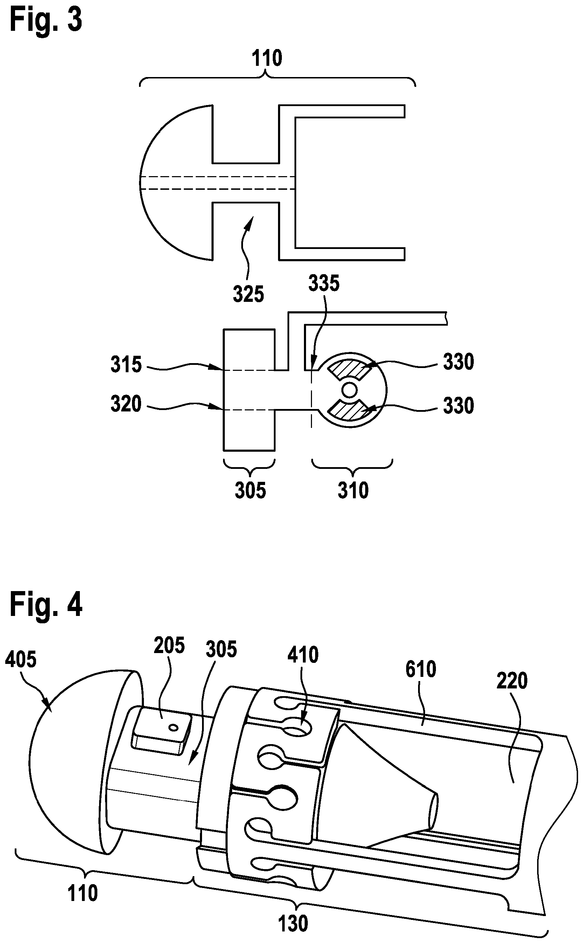

Fig. 3 zeigt eine schematische Ansicht eines Sensorkontaktbereichs 305 sowie eines Signalgeberkontaktierbereichs 310 eines elektrischen Leitelements gemäß einem Ausführungsbeispiel.

Gemäß einem Ausführungsbeispiel weist das elektrische Leitelement beispielhaft an zumindest einem seiner Enden eine Struktur auf, die als Sensorkontaktbereich 305 zur direkten Montage und/oder Kontaktierung von zumindest einem Sensor und/oder als Signalgeberkontaktierbereich 310 zur Kontaktierung zumindest eines Signalgebers durch Leitkleben, Löten und/oder Bonden dient. Der Sensorkontaktbereich 305 und der Signalgeberkontaktierbereich 310 sind hierbei auf der Sensorkopfeinheit 110 angeordnet, die beispielsweise der Druck- und/oder der Temperaturmessung eines herzkranken Patienten dient. Der Sensorkontaktbereich 305 ist beispielhaft als eine rechteckige Leiterplatte ausgebildet, um zumindest zwei Sensoren aufzunehmen bzw. zu kontaktieren. Ferner weist der Sensorkontaktbereich zwei Kanten 315 und 320 auf, wobei der Sensorkontaktbereich 305 an diesen zwei Kanten 315 und 320 gebogen werden kann, um sich um eine Nut 325 in der Sensorkopfeinheit 310 zu legen. Der Signalgeberkontaktierbereich 310 ist beispielhaft als eine kreisrunde Leiterplatte ausgebildet, um zumindest einen Signalgeber, beispielsweise ein Ultraschallelement, aufzunehmen bzw. zu kontaktieren. Hierfür weist der Signalgeberkontaktierbereich 310 gemäß einem Ausführungsbeispiel zwei gebogene Kontaktstellen 330 auf, um das Ultraschallelement zu kontaktieren. Ferner weist der Signalgeberkontaktierbereich 310 ebenfalls eine Kante 335 auf, an der er gebogen werden kann, um sich optimal in die zylinderförmige Ausformung des Herzunterstützungssystems zu integrieren.

Fig. 4 zeigt eine dreidimensionale Ansicht einer Sensorkopfeinheit 110 eines linksventrikulären Herzunterstützungssystems gemäß einem Ausführungsbeispiel. Die Sensorkopfeinheit 110 weist einen beispielhaft pilzförmig ausgeführten Sensorkopf 405 auf, ferner den Sensorkontaktbereich 305, auf dem ein Sensor 205 montiert ist, sowie schließlich das Verbindungselement 130 auf, das eine Mehrzahl von Zulauffenstern 220 aufweist, über die das Blut des herzkranken Patienten in das Herzunterstützungssystem eintritt. Ferner ist ein Anbindungsbereich 410 vorgesehen, welcher der Presspassung des Verbindungselements 130 an die Sensorkopfeinheit 110 dient. Die Zulauffenster 220 sind durch drei Stege 610, von denen zwei in der Fig. 4 auf der rechten Seite sichtbar sind, begrenzt. Um einen möglichen Druckverlust zu minimieren, sind die Zulauffenster größtmöglich ausgeführt, sodass im Bereich 130 die dünnen Stege 610 bestehen bleiben. Wie in der hier gezeigten schematischen Ansicht einer Sensorkopfeinheit 110 dargestellt, ist die Sensorkopfeinheit 110 über das Verbindungselement 130 mit der Leitungsvorrichtung 105 fluiddicht verbunden.

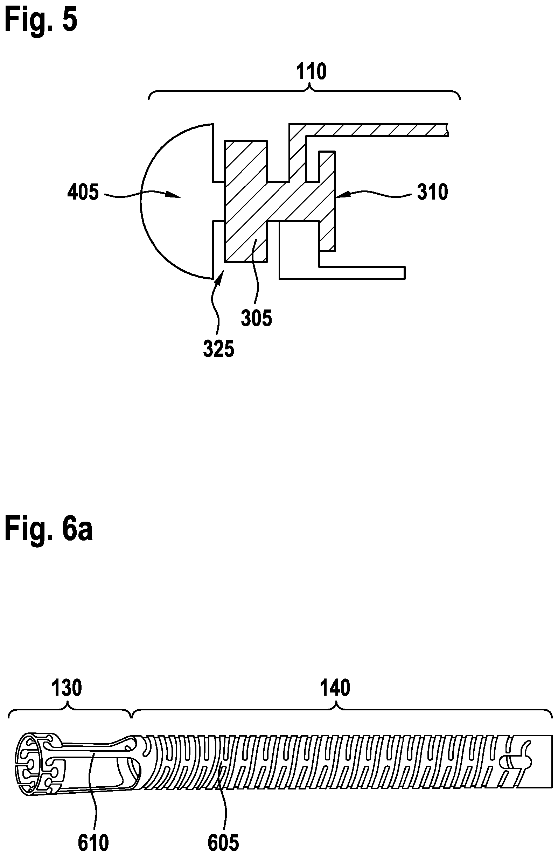

Fig. 5 zeigt eine schematische Ansicht einer Sensorkopfeinheit 110 eines linksventrikulären Herzunterstützungssystems gemäß einem Ausführungsbeispiel. Gemäß einem Ausführungsbeispiel weist die Sensorkopfeinheit 110 einen beispielhaft pilzförmig ausgeführten Sensorkopf 405 auf, ferner den Sensorkontaktbereich 305, auf dem zumindest ein Sensor montiert bzw. kontaktiert ist sowie weiterhin den Signalgeberkontaktierbereich 310, auf dem zumindest ein Signalgeber kontaktiert ist. In der hier gezeigten schematischen Ansicht der Sensorkopfeinheit 110 ist eine Einpassung des Sensorkontaktbereichs 305 und/oder des Signalgeberkontaktierbereichs des elektrischen Leitelements in der Sensorkopfeinheit 110 dargestellt, wobei der Sensorkontaktbereich 305 an seinen zumindest zwei Kanten gebogen ist, um sich um die Nut 325 der Sensorkopfeinheit 110 zu legen. So ist ferner auch der Signalgeberkontaktierbereich 310 an seiner Kante gebogen, um sich optimal in die zylinderförmige Ausformung des Herzunterstützungssystems zu integrieren.

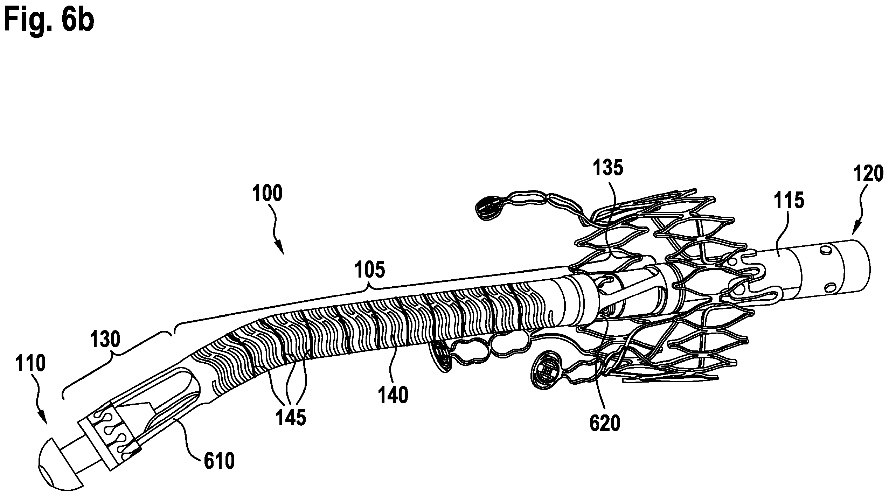

Fig. 6 zeigt eine schematische Ansicht einer Führungskanüle 140 einer Leitungsvorrichtung gemäß einem Ausführungsbeispiel.

Gemäß einem Ausführungsbeispiel ist die Zulaufkanüle 140 als eine Art flexibler zylinderförmiger Zulaufschlauch mit einer durchgängigen strukturierten Oberfläche 605, zur Führung des elektrischen Leitelements ausgeformt.

Hierbei ist die flexible Führungskanüle 140 beispielhaft als eine aus einem Rohr geschnittene Struktur ausgeführt, die einen konstanten Außendurchmesser aufweist, wobei das Schnittmuster eine durchgängige Spirale 605 zur Stützung und zum Schutz des elektrischen Leitelements enthält. Die Führungskanüle weist beispielsweise ferner ein integriertes Verbindungselement 130 auf, bestehend aus einem Anbindungsbereich 410 und mehreren Stegen 610 als Übergangsbereich, mit denen die Führungskanüle 140 mit dem Verbindungselement 130 einstückig verbunden ist, wobei das Verbindungselement 130 beispielhaft aus demselben Material wie einer metallischen Legierung, gefertigt ist. Der Bereich des Verbindungselements 130 zwischen dem Anbindungsbereich 410 und der Führungskanüle bildet die Zulauffenster, die durch dünne Stege 610 (an welchen das elektrische Leitelement 145 geführt werden kann) voneinander separiert sind und über die das Blut des herzkranken Patienten in das Herzunterstützungssystem eintritt.

In einem alternativen Ausführungsbeispiel der Führungskanüle 140, ist diese als ein Geflecht ausgeformt, in dem zum Schutz des elektrischen Leitelements beispielsweise ein Flachband als Träger des elektrischen Leitelements integriert ist. Dieses kann ebenfalls eine metallische Legierung, beispielsweise eine Nickel-Titan-Legierung, aufweisen.

Fig. 6B zeigt eine schematische Darstellung eines Herzunterstützungssystems 100 mit einer Leitungsvorrichtung 105. Erkennbar ist hierbei der Sensorkopf 110 und die beispielsweise aus NiTiNol-Material gefertigte Führungskanüle 140, welche das Verbindungselement 130 und die Leitungsvorrichtung 105 umfasst. Vorne im Bereich des Verbindungselementes 130 läuft das Blut im im Patienten eingesetzten Zustand zwischen den Stegen 610 ins Herzunterstützungssystem 100 ein. Das Verbindungselement 135 ist beispielsweise aus Fertigungsgründen hier als Einzelteil ausgeführt. Das Motorgehäuse 115 und die Endeinheit 120 (welche beispielsweise in der Fig. 6B nicht dargestellte Kontaktstifte zur Kontaktierung eines Kabels durch den Körper des Patienten enthalten kann) sind hinten am Motorgehäuse 112 hermetisch miteinander verschweißt bzw, fluiddicht miteinander verbunden. Das elektrische Leitelement 145 wird außen auf dem Device bzw. der Führungkanüle 140 von der Spitze des Sensorkopfes 110 über die Stege 610 des Einlaufkäfigs bzw. des Verbindungselementes 130 und die Spirale als Teil der Führungskanüle 140 zum Laufradkäfig bzw. dem weiteren Verbindungselement 135, dort über einen dem Steg 610 entsprechenden weiteren Steg 620, dann über den Motor bzw. das Motorgehäuse 115 bis zur Endeinheit 120 geführt, an der das elektrische Leitelement 145 dann mit der Leitung 125 elektrisch kontaktiert werden kann.

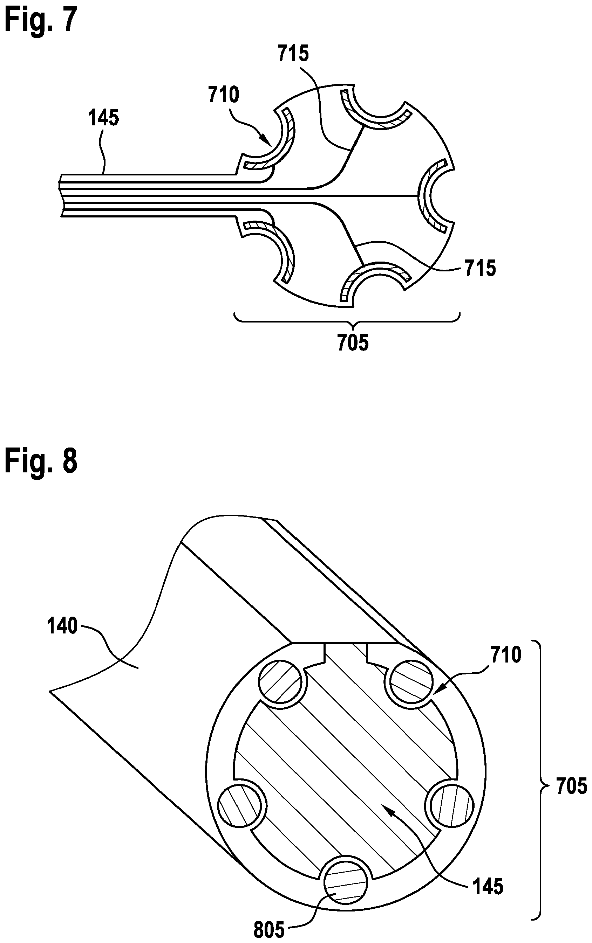

Fig. 7 zeigt eine schematische Ansicht eines Anschlussstellenelements 705 eines elektrischen Leitelements 145 gemäß einem Ausführungsbeispiel.

Gemäß einem Ausführungsbeispiel weist das elektrische Leitelement 145 an einem seiner Enden das Anschlussstellenelement 705 auf. Hierbei ist das Anschlussstellenelement 705 beispielhaft kreisförmig ausgeformt, kann in einem alternativen Ausführungsbeispiel allerdings auch O- oder U-förmig, sechseckig, viereckig, dreieckig oder allgemein polygonförmig ausgeformt sein. Das Anschlussstellenelement 705 weist gemäß einem Ausführungsbeispiel ferner eine Mehrzahl von Verbindungsstellen 710 auf, die an einer Außenumgebung des Anschlussstellenelements 705 radial und/oder umlaufend angeordnet sind. Die Verbindungsstellen 710 sind hierbei rund bzw. halbkreisförmig ausgeführt, sodass das eine Mehrzahl dünner und flexibler Leitungen 715 des elektrischen Leitelements 145 zwischen die ebenfalls radial angeordneten Kontaktstifte eines Durchführungselements (nicht dargestellt) geklappt werden kann, um dort mittels Schweißen, Leitkleben und/oder Löten elektrisch kontaktiert zu werden.

Fig. 8 zeigt eine schematische Ansicht eines kontaktierten Anschlussstellenelements 705 eines elektrischen Leitelements 145 gemäß einem Ausführungsbeispiel an das proximale Ende 120 eines Herzunterstützungssystems. Gemäß einem Ausführungsbeispiel handelt es sich bei dem dargestellten Anschlussstellenelement 705 um eine fertig kontaktierte Anschlussstelle des elektrischen Leitelements 145. Hierbei ist in dieser Ansicht der Mehrschichtaufbau des elektrischen Leitelements 145 gut sichtbar. Das Anschlussstellenelement 705 weist eine Mehrzahl von halbkreisförmigen Verbindungsstellen 710 auf, die an einer Außenumgebung des Anschlussstellenelements 705 radial und/oder umlaufend angeordnet sind. Jede Verbindungsstelle 710 ist hierbei mit je einem Kontaktstift 805 eines Durchführungselements (nicht dargestellt) am proximalen Ende 120 des Unterstützungssystems verbunden, wobei es sich bei dem Durchführungselement ebenfalls um ein Leiterelement handelt, das verwendet wird, um beispielsweise die Energieversorgungsleitungen des Elektromotors 115 an das Zuleitungskabel 125 zu verbinden. Die Kontaktierung des elektrischen Leitelements 145 zunächst an die metallischen Stifte des Backends 120 ermöglicht dabei eine robuste mechanische Ankopplung als gemeinsames Verbindungselement der flexiblen Leiter des Anschlusskabels 125 und des elektrischen Leitelements. Eine direkte Verbindung der Leiter des Anschlusskabels an das elektrische Leitelement ist mechanisch nicht zu empfehlen.

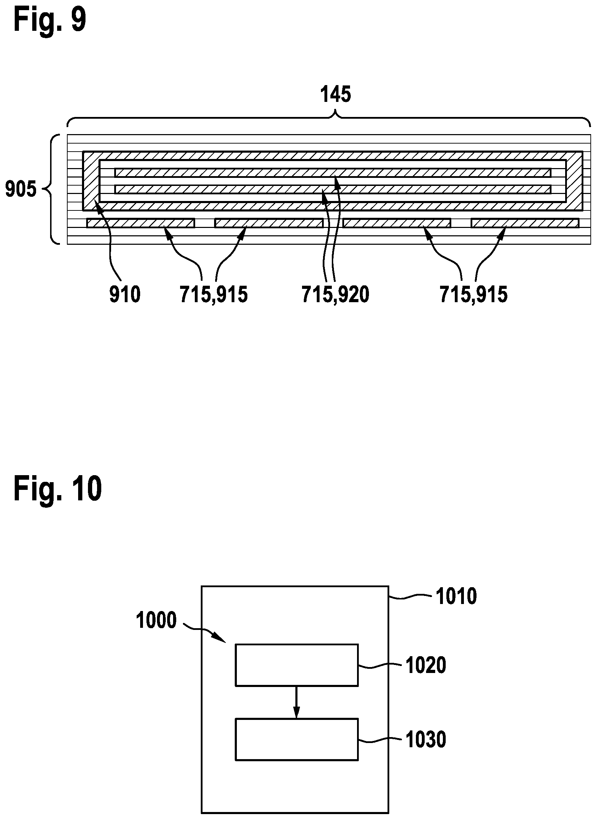

Fig. 9 zeigt eine schematische Querschnittsansicht eines elektrischen Leitelements 145 gemäß einem Ausführungsbeispiel.

Gemäß einem Ausführungsbeispiel weist das elektrische Leitelement 145 eine Mehrzahl koplanarer Schichten 905 aus einem leitenden und/oder isolierenden Material auf. Hierbei weisen die leitenden Schichten beispielhaft zumindest teilweise ein Goldmaterial auf und die isolierenden Schichten sind beispielhaft zumindest teilweise aus einem Polyimid-Material gefertigt. Das elektrische Leitelement 145 weist ferner ein Schirmelement 910 auf, das beispielhaft unter Verwendung der leitenden Schichten realisiert ist und auf einem Goldmaterial basiert. Das Schirmelement 910 weist beispielhaft eine Breite von 470 µm und eine Dicke von 10µm auf. Alternativ zu dem hier gezeigten Ausführungsbeispiel eines Schirmelements 910, kann das Schirmelement 910 ebenfalls unter Verwendung einer Durchkontaktierung zwischen den einzelnen Schichten realisiert sein. Das Schirmelement 910 dient hierbei der Abschirmung einzelner Leiter oder Leiterpaare des elektrischen Verbindungselements, um insbesondere bei höheren Frequenzen möglicherweise auftretende elektrische und/oder magnetische Einkopplung von Feldern in die Leitungen des elektrischen Verbindungselements zu verhindern, oder umgekehrt die elektromagnetische Abstrahlung aus dem elektrischen Verbindungselement zu reduzieren. Darüber hinaus kann die Abschirmung dazu eingesetzt werden, aus Gründen der Hochfrequenztauglichkeit einen gezielten Wellenwiderstand des elektrischen Verbindungselements einzustellen und den Einfluss der Umgebung zu reduzieren.

Das elektrische Leitelement 145 weist weiterhin eine Mehrzahl von Leitungen 715 auf, wobei diese Leitungen 715 innerhalb einer Schicht angeordnet sind und beispielhaft eine Breite von 410 µm und eine Dicke von 10µm aufweisen. So weist das elektrische Leitelement 145 gemäß einem Ausführungsbeispiel vier digitale Leitungen 915 auf, die außerhalb des Schirmelements 910 angeordnet sind, und ferner zwei Ultraschall-Leitungen 920, die innerhalb des Schirmelements 910 angeordnet sind.

Fig. 10 zeigt ein Ablaufdiagramm eines Ausführungsbeispiels eines Verfahrens 1000 zum Herstellen einer Leitungsvorrichtung gemäß einem Ausführungsbeispiel. Gemäß einem Ausführungsbeispiel wird das Verfahren 1000 auf einer Vorrichtung 1010 zum Herstellen einer Leitungsvorrichtung ausgeführt und/oder angesteuert.

In einem Schritt 1020 werden eine Führungskanüle sowie ein elektrisches Leitelement bereitgestellt. In einem Schritt 1030 des Verfahrens 1000 wird das elektrische Leitelement innerhalb der Führungskanüle angeordnet, um eine Leitungsvorrichtung herzustellen.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Leitungsvorrichtung (105) für ein Herzunterstützungssystem (100), wobei die Leitungsvorrichtung (105) folgende Merkmale aufweist:
eine zumindest teilweise entlang einer Erstreckungsrichtung strukturierte Führungskanüle (140), die Strukturen in einem Mantel aufweist, und
ein gesondert von der Führungskanüle bereitgestelltes elektrisches Leitelement (145), das in, auf oder an den Strukturen in dem Mantel der Führungskanüle (140) angeordnet ist, wobei das elektrische Leitelement (145) eine Mehrschichtstruktur aufweist, **dadurch gekennzeichnet dass** das elektrische Leitelement (145) zur direkten Montage und/oder
Kontaktierung zumindest eines Sensors (205) einen Sensorkontaktbereich (305) aufweist.

2. Leitungsvorrichtung (105) gemäß Anspruch 1, bei dem das elektrische Leitelement (145) eine Mehrzahl von Schichten (905) aus einem leitenden und/oder isolierenden Material aufweist, insbesondere wobei eine leitende Schicht zumindest teilweise ein Goldmaterial aufweist und/oder eine isolierende Schicht zumindest teilweise aus einem Polyimid-Material gefertigt ist.

3. Leitungsvorrichtung (105) gemäß einem der vorangegangenen Ansprüche, bei dem das elektrische Leitelement (145) ein Schirmelement (910) aufweist, insbesondere wobei das Schirmelement (910) unter Verwendung der leitenden Schichten und/oder einer Durchkontaktierung zwischen den einzelnen Schichten realisiert ist.

4. Leitungsvorrichtung (105) gemäß einem der vorangegangenen Ansprüche, bei dem das elektrische Leitelement (145) eine Mehrzahl von Leitungen (715) aufweist, wobei die Leitungen (715) innerhalb einer Schicht angeordnet sind, insbesondere wobei die Mehrzahl von Leitungen (715) außerhalb des Schirmelements (910) angeordnet ist.

5. Leitungsvorrichtung (105) gemäß einem der vorangegangenen Ansprüche, bei der das elektrische Leitelement (145) zur Kontaktierung zumindest eines Signalgebers (210) einen Signalgeberkontaktierbereich (310) aufweist.

6. Leitungsvorrichtung (105) gemäß einem der vorangegangenen Ansprüche, bei dem der Sensorkontaktbereich (305) ausgebildet ist, zumindest zwei Sensoren (205) aufzunehmen und/oder zu kontaktieren und/oder der Sensorkontaktbereich (305) rechteckig ausgeformt, insbesondere wobei der Sensorkontaktbereich (305) zumindest zwei Kanten (315, 320) aufweist, wobei der Sensorkontaktbereich (305) an den zumindest zwei Kanten (315, 320) gebogen ist.

7. Leitungsvorrichtung (105) gemäß Anspruch 5 oder 6, bei dem der Signalgeberkontaktierbereich (310) zumindest zwei gebogene Kontaktstellen (330) aufweist.

8. Leitungsvorrichtung (105) gemäß einem der vorangegangenen Ansprüche, bei der das elektrische Leitelement (145) ein Anschlussstellenelement (705) aufweist, wobei das Anschlussstellenelement (705) kreisförmig, sechseckig, viereckig, dreieckig, allgemein polygonförmig, O-förmig oder U-förmig ausgeformt ist, insbesondere wobei das Anschlussstellenelement (705) eine Mehrzahl von runden Verbindungsstellen (710) und/oder an einer Außenumgebung des Anschlusselements (705) radial und/oder umlaufend angeordnete Verbindungsstellen (710) aufweist.

9. Leitungsvorrichtung (105) gemäß einem der vorangegangenen Ansprüche, bei der ein vorzugsweise zylindrischer Mantel der Führungskanüle (140) als ein Geflecht und/oder als eine aus einem Rohr geschnittene Spiral- oder Wellenstruktur oder als gezackte Struktur ausgeformt ist, insbesondere wobei der Mantel der Führungskanüle (140) eine metallhaltige Legierung aufweist.

10. Leitungsvorrichtung (105) gemäß einem der vorangegangenen Ansprüche, bei der je eine Schicht des elektrischen Leitelements (145) eine Dicke in einem Bereich zwischen 5 µm und 15 µm aufweist und/oder wobei das elektrische Leitelement (145) mäanderförmig ausgeformt ist.

11. Herzunterstützungssystem (100) mit einer Leitungsvorrichtung (105) gemäß einem der vorangegangenen Ansprüche, wobei die Leitungsvorrichtung (105) zwischen einer Sensorkopfeinheit (110) und einer Endeinheit (120) des Herzunterstützungssystems (100) angeordnet ist, insbesondere wobei zwischen der Leitungsvorrichtung (105) und der Sensorkopfeinheit (110) und/oder zwischen der Leitungsvorrichtung (105) und der Endeinheit (120) je ein Verbindungselement (130, 135) angeordnet oder ausgebildet ist.

12. Verfahren (1000) zum Herstellen einer Leitungsvorrichtung (105) gemäß einem der Ansprüche 1-10, wobei das Verfahren (1000) die folgenden Schritte aufweist:
Bereitstellen (1020) der Führungskanüle (140), die Strukturen in einem Mantel aufweist, sowie des elektrischen Leitelements (145), das zur direkten Montage und/oder Kontaktierung zumindest eines Sensors (205) einen Sensorkontaktbereich (305) aufweist; und
Anordnen (1030) des elektrischen Leitelements (145) innerhalb oder an den Strukturen in dem Mantel der Führungskanüle (140), um die Leitungsvorrichtung (105) herzustellen.

13. Vorrichtung (1010), die eingerichtet ist, um die Schritte (1020, 1030) des Verfahrens (1000) gemäß Anspruch 12 in entsprechenden Einheiten auszuführen und/oder anzusteuern.

14. Computerprogramm, das dazu eingerichtet ist, die Schritte des Verfahrens (1000) gemäß Anspruch 12 auszuführen und/oder anzusteuern.

15. Maschinenlesbares Speichermedium, auf dem das Computerprogramm nach Anspruch 14 gespeichert ist.

## Claims

1. Line device (105) for a cardiac support system (100), the line device (105) having the following features:
a guide cannula (140) which is structured at least partially along a direction of extension and has structures in a sheath, and
an electrical conducting element (145) which is provided separately from the guide cannula and which is arranged in, on or at the structures in the sheath of the guide cannula (140), the electrical conducting element (145) having a multi-layer structure, **characterized in that** the electrical conducting element (145) has a sensor contact region (305) for directly mounting and/or contacting at least one sensor (205).

2. Line device (105) according to claim 1, wherein the electrical conducting element (145) has a plurality of layers (905) made of a conductive and/or insulating material, in particular wherein a conductive layer at least partially comprises a gold material and/or an insulating layer is at least partially made of a polyimide material.

3. Line device (105) according to any of the preceding claims, wherein the electrical conducting element (145) has a shielding element (910), in particular wherein the shielding element (910) is produced using the conductive layers and/or a through-connection between the individual layers.

4. Line device (105) according to any of the preceding claims, wherein the electrical conducting element (145) has a plurality of lines (715), wherein the lines (715) are arranged within a layer, in particular wherein the plurality of lines (715) is arranged outside the shielding element (910).

5. Line device (105) according to any of the preceding claims, wherein the electrical conducting element (145) has a signal generator contact region (310) for contacting at least one signal generator (210).

6. Line device (105) according to any of the preceding claims, wherein the sensor contact region (305) is designed to accommodate and/or contact at least two sensors (205) and/or the sensor contact region (305) is rectangular in shape, in particular wherein the sensor contact region (305) has at least two edges (315, 320), wherein the sensor contact region (305) is curved at the at least two edges (315, 320).

7. Line device (105) according to either claim 5 or claim 6, wherein the signal generator contact region (310) has at least two curved contact points (330).

8. Line device (105) according to any of the preceding claims, wherein the electrical conducting element (145) has a connection point element (705), wherein the connection point element (705) is circular, hexagonal, square, triangular, generally polygonal, O-shaped or U-shaped, in particular wherein the connection point element (705) has a plurality of round connection points (710) and/or connection points (710) which are arranged radially and/or circumferentially on an outer periphery of the connection element (705).

9. Line device (105) according to any of the preceding claims, wherein a preferably cylindrical sheath of the guide cannula (140) is formed as a braid and/or as a spiral structure or wave structure cut from a tube or as a serrated structure, in particular wherein the sheath of the guide cannula (140) comprises a metal-containing alloy.

10. Line device (105) according to any of the preceding claims, wherein each layer of the electrical conducting element (145) has a thickness in a range between 5 µm and 15 µm and/or wherein the electrical conducting element (145) has a meandering shape.

11. Cardiac support system (100) comprising a line device (105) according to any of the preceding claims, wherein the line device (105) is arranged between a sensor head unit (110) and an end unit (120) of the cardiac support system (100), in particular wherein a connecting element (130, 135) is arranged or formed in each case between the line device (105) and the sensor head unit (110) and/or between the line device (105) and the end unit (120).

12. Method (1000) for manufacturing a line device (105) according to any of claims 1-10, wherein the method (1000) comprises the following steps:
providing (1020) the guide cannula (140), which has structures in a sheath, and the electrical conducting element (145), which has a sensor contact region (305) for directly mounting and/or contacting at least one sensor (205); and
arranging (1030) the electrical conducting element (145) within or on the structures in the sheath of the guide cannula (140) in order to produce the line device (105).

13. Device (1010) designed to carry out and/or control the steps (1020, 1030) of the method (1000) according to claim 12 in corresponding units.

14. Computer program designed to carry out and/or control the steps of the method (1000) according to claim 12.

15. Machine-readable storage medium on which the computer program according to claim 14 is stored.

## Revendications

1. Dispositif de ligne électrique (105) pour système d'assistance ventriculaire (100), dans lequel le dispositif de ligne électrique (105) présente les caractéristiques suivantes :
une canule de guidage (140) structurée au moins partiellement le long d'une direction d'extension et qui présente des structures dans une enveloppe, et
un élément conducteur électrique (145) fourni séparément de la canule de guidage et qui est disposé dans, sur ou au niveau des structures dans l'enveloppe de la canule de guidage (140), l'élément conducteur électrique (145) présentant une structure multicouche, **caractérisé en ce que** l'élément conducteur électrique (145) présente une zone de contact de capteur (305) pour le montage direct et/ou la mise en contact d'au moins un capteur (205).

2. Dispositif de ligne électrique (105) selon la revendication 1, dans lequel l'élément conducteur électrique (145) présente une pluralité de couches (905) en un matériau conducteur et/ou isolant, en particulier une couche conductrice présentant au moins partiellement un matériau en or et/ou une couche isolante étant fabriquée au moins partiellement à partir d'un matériau polyimide.

3. Dispositif de ligne électrique (105) selon l'une des revendications précédentes, dans lequel l'élément conducteur électrique (145) présente un élément de blindage (910), en particulier l'élément de blindage (910) étant réalisé à l'aide des couches conductrices et/ou d'une métallisation des trous entre les couches individuelles.

4. Dispositif de ligne électrique (105) selon l'une des revendications précédentes, dans lequel l'élément conducteur électrique (145) présente une pluralité de lignes électriques (715), les lignes électriques (715) étant disposées à l'intérieur d'une couche, en particulier la pluralité de lignes électriques (715) étant disposées à l'extérieur de l'élément de blindage (910).

5. Dispositif de ligne électrique (105) selon l'une des revendications précédentes, dans lequel l'élément conducteur électrique (145) présente une zone de mise en contact d'émetteur de signal (310) permettant la mise en contact d'au moins un émetteur de signal (210).

6. Dispositif de ligne électrique (105) selon l'une des revendications précédentes, dans lequel la zone de contact de capteur (305) est conçue pour recevoir et/ou mettre en contact au moins deux capteurs (205) et/ou la zone de contact de capteur (305) est rectangulaire, en particulier la zone de contact de capteur (305) présentant au moins deux bords (315, 320), la zone de contact de capteur (305) étant incurvée au niveau des au moins deux bords (315, 320).

7. Dispositif de ligne électrique (105) selon la revendication 5 ou 6, dans lequel la zone de mise en contact d'émetteur de signal (310) présente au moins deux points de contact (330) incurvés.

8. Dispositif de ligne électrique (105) selon l'une des revendications précédentes, dans lequel l'élément conducteur électrique (145) présente un élément de point de connexion (705), l'élément de point de connexion (705) étant circulaire, hexagonal, quadrangulaire, triangulaire, généralement polygonal, en forme de O ou en forme de U, en particulier l'élément de point de connexion (705) présentant une pluralité de points de jonction (710) ronds et/ou de points de jonction (710) disposés radialement et/ou circonférentiellement sur une zone extérieure de l'élément de connexion (705).

9. Dispositif de ligne électrique (105) selon l'une des revendications précédentes, dans lequel une enveloppe de préférence cylindrique de la canule de guidage (140) est réalisée sous forme de tresse et/ou de structure spiralée ou ondulée découpée dans un tube ou de structure dentelée, en particulier l'enveloppe de la canule de guidage (140) présentant un alliage contenant du métal.

10. Dispositif de ligne électrique (105) selon l'une des revendications précédentes, dans lequel chaque couche de l'élément conducteur électrique (145) présente une épaisseur dans une plage comprise entre 5 µm et 15 µm et/ou l'élément conducteur électrique (145) étant en forme de méandres.

11. Système d'assistance ventriculaire (100) comportant un dispositif de ligne électrique (105) selon l'une des revendications précédentes, dans lequel le dispositif de ligne électrique (105) est disposé entre une unité de tête de capteur (110) et une unité d'extrémité (120) du système d'assistance ventriculaire (100), en particulier un élément de jonction (130, 135) étant disposé ou formé entre le dispositif de ligne électrique (105) et l'unité de tête de capteur (110) et/ou entre le dispositif de ligne électrique (105) et l'unité d'extrémité (120).

12. Procédé (1000) de fabrication d'un dispositif de ligne électrique (105) selon l'une des revendications 1 à 10, dans lequel le procédé (1000) présente les étapes suivantes :
fourniture (1020) de la canule de guidage (140) présentant des structures dans une enveloppe, et de l'élément conducteur électrique (145) présentant une zone de contact de capteur (305) pour le montage direct et/ou la mise en contact d'au moins un capteur (205) ; et
disposition (1030) de l'élément conducteur électrique (145) à l'intérieur ou sur des structures dans l'enveloppe de la canule de guidage (140) pour fabriquer le dispositif de ligne électrique (105).

13. Dispositif (1010) configuré pour exécuter et/ou commander les étapes (1020, 1030) du procédé (1000) selon la revendication 12 dans des unités correspondantes.

14. Programme informatique configuré pour exécuter et/ou commander les étapes du procédé (1000) selon la revendication 12.

15. Support de stockage lisible par machine sur lequel est stocké le programme informatique selon la revendication 14.
